# NEW EUROPEAN PATENT SPECIFICATION

(11) **EP 1 294 371 B2**
(45) Date of publication and mention of the opposition decision: **26.05.2010**
(45) Mention of the grant of the patent: 15.12.2004
(21) Application number: 01965011.8
(22) Date of filing: 20.06.2001
(51) Int. Cl.: A61K 31/20, A61K 31/19, A61K 31/215, A61K 31/23, A23K 1/16, A23L 3/34, A61P 31/00

(54) **MEDIUM CHAIN FATTY ACIDS APPLICABLE AS ANTIMICROBIAL AGENTS**
MITTELKETTIGE FETTSÄUREN UND DEREN VERWENDUNG ALS ANTIMIKROBIELLES MITTEL
ACIDES GRAS A CHAINES MOYENNES UTILISABLES COMME AGENTS ANTIMICROBIENS

(30) Priority: 20.06.2000 EP 00870137
(43) Date of publication of application: 26.03.2003
(73) Proprietor: Nutrition Sciences, 9031 Drongen (BE)
(72) Inventor: MOLLY, Koen, B-9870 Olsene (BE); BRUGGEMAN, Geert, B-8310 Brugge (BE)
(74) Representative: Kraft, Henricus Johannes
(86) International application number: PCT/EP2001/006973
(87) International publication number: WO 2001/097799

(56) References cited:
- EP-A- 0 089 376
- EP-A- 0 519 458
- WO-A1-00/36928
- US-A- 3 658 548
- US-A- 4 961 934
- US-A- 6 033 705
- PETSCHOW, B. W., BATEMA, R. P., TALBOTT, R. D., AND FORD, L. L.: "Impact of medium-chain monoglycerides on intestinal colonisation by Vibrio cholerae or enterotoxigenic Escherichia coli" JOURNAL OF MEDICAL MICROBIOLOGY., vol. 47, 1998, pages 383-389, XP000960797 HARLOW, GB ISSN: 0022-2615
- KINDERLERER, J. L., MATTHIAS, H. E., AND FINNER, P.: "Effect of medium chain fatty acids in mould ripened cheese on the growth of listeria monocytogenes" JOURNAL DAIRY RESEARCH, vol. 63, no. 4, 1996, pages 593-606, XP000960914 CAMBRIDGE, GB

## Description

### Technical field

The present invention relates to the use of a specific range within the medium chain fatty acids (MCFA) as inhibitors of microbial, in particular bacterial and fungal contamination and growth. In particular the invention relates to the use of caprylic (C₈), and capric (C₁₀) acid or mixtures or emulsions thereof to inhibit bacterial and fungal contamination and growth and where appropriate for the inhibition of the subsequent toxin production by these microbial organisms.

More in particular the invention relates to a mixture comprising essentially equal amounts in weight of C₈ and C₁₀ as antimicrobial agents, being mainly active in acidic environments such as the stomach.

### Background of the invention

Micro-organisms are the biggest source of food and feed spoilage and Involve a considerable loss of essential nutrients (Bartov et al., 1982). All food and feed ingredients are naturally contaminated with bacteria, yeast and (mainly mould-forming) fungi, the last usually in the form of spores. At temperatures above 4°C, most foods and feeds are ideal media for microbial growth and most of the time for a subsequent toxic metabolite (endotoxin, mycotoxin, ...) production (Smith et al., 1983; Russel et al., 1991) by the in situ development of micro-organisms.

In this context, contamination of agricultural products with fungi is a very illustrative example. Contamination with mycotoxin producing fungi is often unavoidable and of world-wide concern (Tuite, 1979; Jelinek at al., 1989). Moreover, since humidity and temperature are important parameters for fungal growth, liquid food and feed are very susceptible to fungal contamination and growth and subsequent mycotoxin presence.

Although some moulds and fungi are non-toxic and cause little difficulty even allowed to multiply, others cause considerable troubles. Moreover, under standard management practices it is impossible to differentiate between the non-toxic and toxic form of moulds and fungi (Hirooka et al., 1996).

Fungi and moulds that cause diseases in humans and animals, can do this by direct infection (mycosis) or by producing mycotoxins, which become further ingested (mycotoxicosis) (Wyatt, 1995). Examples of mycosis are Aspergillosis in poultry, Fog Fever in cattle and Farmers Lung. These diseases are sometimes very difficult to cure. At the other side, mycotoxicosis received world-wide attention in the early 1960's when about one million birds (turkey's) died in the UK due to aflatoxicosis (mycotoxicosis by aflatoxins). Mycotoxicosis can be caused by following mycotoxins: aflatoxins, ochratoxins, trichothecenes, zearalenones, citrinins, ... .

Mycotoxins cause a wide variety of adverse clinical signs depending on the nature and concentration of mycotoxins present, duration of exposure, the animal species, its age and nutritional health status at the time of exposure to the contaminated food and feed (Reiss, 1978; Bartov et al., 1982; Harvey et al., 1989; Hamilton, 1990; Pier, 1992). Mould growth and subsequent mycotoxin production result from a wide variety of plant and environmental factors affecting the ability of fungi to invade and colonise plant parts. For this reason, in the past, several techniques were applied in order to minimise fungal colonisation, contamination and growth and subsequent mycotoxin production.

Perhaps among the most ancient of these preservative systems for fungal contamination of foods and feeds, were the processes of air-drying, sun-curing, drying by artificial means, smoking, storage under anaerobic conditions, fermentation, pickling, salting, sugaring and the addition of spices to certain foods and feeds. However, these traditional methods are relatively costly and very time-consuming. Additionally, these methods are not really useful if the food- and feedstuffs are in transit or have been mixed with other ingredients in a complete diet.

Therefore, other means such as chemical preservation became in the last decades a more attractive alternative. Chemical fungal preservation of (high moisture) foods and feeds and their feeding to humans and livestock are well documented (Jones et al., 1970; English et al., 1973; Foster et al., 1987; Yasin and Hanna, 1989). But nowadays, storing without delay, keeping moisture as low as possible and the addition of mould inhibitors (such as propionate) or other chemical preservatives (for example acid inhibitors, such as benzoic, sorbic, acetic acids and their salts) or derivatives thereof or mixtures thereof are generally applied in order to diminish fungal growth in food and feed stuffs (Smith et al., 1983).

Propionic acid and all traditional acid preservatives are very effective mould inhibitors, but because of their volatility and irritative and corrosive nature, these acids have not been used so extensively (Marin et al., 1999). For these reasons, various buffered forms of propionic acid (dry and liquid) are currently being marketed, but only limited and dubious information is available on the efficacy of these types of 'blended' chemicals (Rahnema and Neal, 1992). Some work is done to evaluate these new types of 'blended' chemicals (Rahnema and Neal, 1994). Further on, some non-acid antifungal agents are nowadays available, such as natamycin, nystatin, ... (Rybinska et al., 1997; Emele et al., 2000). But some of these new antifungal agents suffer by ethical, emotional and economic aspects.

Although last centuries, most attention was focussed on microbial contamination in human nutrition, the practice of animal feed preservation has nowadays also become important. Next example illustrates the importance of feed preservation against microbial contamination.

In view of the economical interest of modern animal husbandry systems to increase productivity and maintain profitability, it has become general practice to increase the growth rate by subjecting specific animals (such as piglets,....) to an early weaning. This early weaning however burdens the animal with a lot of adverse stresses, mainly of nutritional origin (Zijlstra et al., 1996). The adverse stresses are often accompanied by a more or less severe decrease in feed intake and energy deficiency and thus involve mobilization of body reserves by the animal. Maldigestion and malabsorption may further aggravate the situation and result in digestive upsets mainly due to bacterial and fungal overgrowth in and/or viral infections of the gastrointestinal tract (Eckel, 1999).

There is a general belief that the digestive pathology of early weaned animals is mainly caused by Gram negative bacteria, in particular Escherichia coli spp. and Salmonella spp., which are often present in the intestine of the animal as such or may enter the gastrointestinal tract through the feed (Guillot, 1989). To overcome the problem of digestive pathology, which often involves a severe weight loss and an increased mortality amongst the animals, it became common practice to supplement the animal feed with low doses of pharmaceutical antimicrobial substances (for example antibiotics) or therapeutic doses of antibiotics, designated as "antibiotics" further on (Dupont and Steele, 1987; Prescott, 1997). Nowadays however, there is a growing concern on the addition of antibiotics to the feed (Guillot, 1989; Barton, 1998). There is a fear for the risk of the emergence of last-resort antibiotics used in human medicine, of the development of a resistance towards the antibiotics, which would involve a need to increase their dosing or to develop new, stronger antibiotics. There is also a fear that a resistance may emerge amongst living beings after consuming animals that have been treated with those antibiotics. The present concern of environmental disturbance by chemicals and the fact that most of those antibiotics have already been banned in the European Union, or will be in the near future, justify the need for alternatives, functional as antimicrobial agents (Muirhead, 1998; Ross, 1999).

As can be derived from the given examples, there is thus a need to find new antimicrobial agents as a substitute for the known antimicrobial agents and for in the mean time sometimes banned antibiotics to overcome microbial (out)growth and to overcome digestive pathology exhibited by animals.

A further object is to provide an antimicrobial feed additive able to be active at low pH values present in the stomach. This avoids further transit of ingested pathogens to the intestines.

In this context, specific medium-chain fatty acids MCFA (C₆, C₇, C₈, C₉ and C₁₀) are a valuable alternative, since they can be used as novel and innovative antimicrobial agents, in order to control microbial contamination and growth and subsequent toxin production in the gastrointestinal tract and more in particular in the stomach.

EP-A1-0 089 376 describes a feed additive or feed of accelerating growth of animals containing at least a fatty acid salt or at least a fatty acid salt and a fatty acid ester of sugar. In said document fatty acids consist of about 6-24 carbons and describes a very broad field of chain lengths. No specific sub-range is provided showing a better effect than the broad range.

EP-A1-0 519 458 describes a feed additive for livestock and a feed for livestock which comprises (a) a triglyceride of a medium-chain fatty acid having 6 to 12 carbon atoms and (b) at least one substance selected from the medium-chain fatty acid having 6 to 12 carbon atoms, a monoglyceride of the fatty acid and a diglyceride of a fatty acid. This composition discloses a combination of fatty acids.

The main object of the present invention is to provide a more specific and more active range of medium-chain fatty acids having improved antimicrobial, in particular combined antibacterial and antifungal properties. It is believed that said specific combination of activities results surprisingly in an effective feed additive resulting in a improved feed conversion ratio (being the weight feed consumed per kg body weight gain).

The invention provides therefor the use of two C₆-C₁₀ medium-chain fatty acids, emulsions or mixtures thereof for inhibition of microbial contamination, growth and subsequent toxin production. Preferably substantially equal amounts of C₈ and C₁₀ are used.

### Description of invention

The present invention describes the use of two medium chain fatty acids (MCFA), chosen from the group consisting of caprylic (C₈) acid and capric (C₁₀) acid, mixtures thereof, about equal amounts by weight, in an amount of: 20-50% C₈, 20-50% C₁₀ and optionally other MCFA chosen from C₆-C₂₄ for the inhibition of microbial contamination, growth and/or the subsequent toxin production.

The invention relates to the use of two medium chain fatty acids (MCFA), chosen from the group consisting of caprylic (C₈) acid and capric (C₁₀) acid, emulsions or mixtures thereof, about equal amounts by weight, in an amount of:
20-50% C₈
20-50% C₁₀
and optionally other MCFA chosen from C₆- C₂₄
wherein the MCFA concentration is 100-3000ppm for the manufacture of a medicament for the inhibition of microbial contamination, growth and/or the subsequent toxin production.

The invention further relates to the use as described above, of caprylic (C₈) acid and capric (C₁₀) acid in about equal amounts by weight and in a concentration of 100-3000ppm for the manufacture of a medicament for the inhibition of microbial contamination, growth and/or the subsequent toxin production.

The invention further relates to the use as described above, in combination with other MCFA, such as lauric (C₁₂) and myristic (C₁₄) acid, other antifungal agents or with other (organic) (fatty) acids or with additives, such as aroma's and plant extracts.

The invention further relates to the use as described above, for combatting the growth of fungi and yeasts chosen from the group: Penicillium, Aspergillus, Fusarium, Cephalosporum, Saccharomyces, Candida as well as to other Fungi Imperfecti and Hemiascomycetes (yeasts).

The invention further relates to the use as described above for selective combatting the growth of Gram negative bacteria such as Escherichia coli, Salmonella sp., Shigella sp. and other Gram negative and coliform bacteria and food/feed spoilers.

The invention further relates to the use as described above for the selective control and regulation of the microbial ecosystem in the gastrointestinal tract of any animal or human.

The invention further relates to the use as described above wherein the animals are in their early weaning phase.

The invention also relates to a feed composition for an animal comprising a feed supplement containing one or more medium chain fatty acids (MCFA) chosen from the group consisting of caprylic (C₈) acid and capric (C₁₀) acid, emulsions or mixtures thereof in an amount by weight % of:
20-50% C₈
20-50% C₁₀
and optionally other MCFA chosen from C₆-C₂₄,
wherein the MCFA concentration is 100-3000ppm.

The invention further relates to a feed composition as described above, containing caprylic (C₈) acid and capric (C₁₀) acid in about equal amounts by weight and in a concentration of 100-3000ppm.

In first instance, the present invention relates to the surprising observation that supplying specific MCFA in the range of C₆-C₁₀, or mixtures as a solution or an emulsion thereof to fungi, yeasts and bacteria, inhibit their further growth. Growth of fungi, yeasts and bacteria is inhibited and the respective micro-organisms are killed by the administered MCFA. In the present invention, preferably use is made of a mixture of specific different fatty acids, the individual fatty acids containing a different number of carbon atoms. The inventors have found that such a mixture shows optimal antimicrobial properties. The fatty acids that can be used in this invention include both fatty acids with an even and an odd number of carbon atoms, for example C₆ (caproic acid, hexanoic acid), C₇ (heptanoic acid), C₈ (caprylic acid, octanoic acid), C₉ (nonanoic or pelargonic acid) and C₁₀ (capric acid, decanoic acid).

The antimicrobial effects of fatty acids and their soaps have already been known for a long time and have been reviewed by J.J. Kabara (1978) in "The pharmacological effects of lipids". In this review it is discussed that in homologous series of fatty acids, the bactericidal efficiency has been found to increase with increasing chain length. E. coil spp. and Shigella spp. appear to be killed by moderate concentrations of saturated soaps of lauric acid containing 12 carbon atoms, and stearyl fatty acid containing 18 carbon atoms. Fatty acids with a chain length of 10 to 12 carbon atoms appear to show optimal antimicrobial activity, whereas lower fatty acids with 4-10 carbon atoms appear to have no or little germicidal effect.

The mechanism according to which the fatty acids exert antimicrobial activity has been documented in literature. The currently accepted theory is that the lipid microbial cell membrane is permeable for the undissociated fatty acid, as a consequence of which the fatty acid is capable of passing across the microbial cell membrane towards the more alkaline interior. Because of the higher intracellular alkalinity, the fatty acid is dissociated, thus involving a decrease of the intracellular pH below the survival level. The fatty acid thus in fact acts as a protonophore, which increases inward leak of H⁺ and involves that efflux of H⁺ is too slow to allow the intracellular pH to be increased again. The physicochemical properties of the fatty acids which allow them to act as protonophores, may vary and depend on numerous parameters. Examples of such parameters are the chain length and pKa of the fatty acid, as well as the physicochemical environment, precipitations, the pH at the place of action and the chemical composition of the microbial envelope which determines the passage of the fatty acids through the membrane. In this respect, the better performance of the fatty acid containing 8-10 carbon atoms according to the invention is attributed to the extreme permeability of the microbial cell membrane for this fatty acid. This is quite unexpected, since Kabara (1978) discloses that the lower fatty acids containing 4-10 carbon atoms show little germicidal activity. An increase of the pH from 6.5 to 7.5 increased the minimum inhibitory concentration of the short chain fatty acids containing 6-8 carbon atoms, and decreased the minimum concentrations of the two medium chained fatty acids containing 12-14 carbon atoms (lauric, myristic acid).

There is however no teaching in Kabara (1978) that fatty acids containing 8-10 carbon atoms would be capable of controlling the bacterial.

This particular antimicrobial action of C₈-C₁₀ MCFA is in particular effective for a combined inhibition against fungi, yeasts and Gram negative bacteria. Fungi can include following genera: Aspergillus, Candida, Cephalosporum, Fusarium, Penicillium as well as other fungi belonging to the Fungi Imperfecti. Yeasts can include, Saccharomyces and other hemiascomycetes (yeasts). Gram negative bacteria include Escherichia coli, Salmonella sp., Shigella sp. and other Gram negative and coliform bacteria and spoilers. By inhibition of growth and by killing the microbial cell, the respective micro-organisms are not able anymore to cause cell intrinsic diseases and to produce further toxins.

From these results and observations by others in the field, it would be expected that other MCFA are also useful for this application. Other MCFA can include lauric acid (C₁₂) and myristic acid (C₁₄). A concentration up to about 100000 ppm MCFA eventually combined with other (organic) (fatty) acids, antimicrobial agents and additives, such as aroma's, ..., can be used to achieve this particular goal. 1200 ppm of a mixture of MCFA has been found to be particularly suitable (see examples).

Conclusive, MCFA (mixtures or emulsions thereof) inhibit microbial growth by killing the microbial cells.

The invention relates in particular to a specific small range of MCFA (C₈-C₉-C₁₀) and more in particular about equal amounts in weight of C₈ and C₁₀ are suitable as antimicrobial agents. As a result, microbial growth during contamination, during biofouling, during fermentation, in ecological systems such as the gastrointestinal tract can be controlled in a more friendly way, compared to the use of traditional antibiotics such as propionic acid, growth promoters and antibiotics.

The observed effect is obtained, with the free MCFA or as an emulsion of the MCFA.

In order that the present invention may be more clearly understood, the preferred form will be described with reference to the following examples.

Possible uses are all situations where microbial contamination is disadvantageous and must be monitored and controlled. For this reason, the specific MCFA can be applied in crop protection on growing, harvested and stored plants, in powdered and liquid foods and feeds, as antifouling agent in tubes and tanks (fluid sanitising), in human healthcare (in case of diarrhoea or as aerosol for respiratory diseases or during topical applications, such as for wound healing and in case of vaginal infection, in case of dermatological diseases, in case of oral diseases, ...), in food and feed preservation (fruit, cheese, cake, bread, ...), in drinks, in cleaning (disinfecting) agents and detergents, ....

Hereunder several examples will prove the effectiveness of the antimicrobial effectiveness of MCFA being a 50/50 by weight mixture of C₈ and C₁₀. It is clear that these examples will have an explanatory goal and are not limitative to the scope of the invention which is worded in the claims.

### Brief description of the drawings

The drawings are schematic and should not be construed as limiting the scope of the invention.
Figure 1 shows the linear relationship between OD₆₀₀ₙₘ and the amount of *E. coli* K88 cells.
Figure 2 shows the *E. coli* counts (as log₁₀ CFU) in the control animals in the different parts of the gastrointestinal tract in function of time (in days) after administration of MCFA's.
Figure 3 shows the *E. coli* counts (as log₁₀ CFU) in the treated animals in the different parts of the gastrointestinal tract in function of time (in days) after administration of MCFA's.
Figure 4 shows the *Enterobacteriaceae* counts (as log₁₀ CFU) in the control animals in the different parts of the gastrointestinal tract in function of time (in days) after administration of MCFA's.
Figure 5 shows the *Enterobacteriaceae* counts (as log₁₀ CFU) in the treated animals in the different parts of the gastrointestinal tract in function of time (in days) after administration of MCFA's.
Figure 6 shows the total counts (as log₁₀ CFU) in the control animals in the different parts of the gastrointestinal tract in function of time (in days) after administration of MCFA's.
Figure 7 shows the total counts (as log₁₀ CFU) in the treated animals in the different parts of the gastrointestinal tract in function of time (in days) after administration of MCFA's.
Figure 8 shows the lactic acid bacteria counts (as log₁₀ CFU) in the control animals in the different parts of the gastrointestinal tract in function of time (in days) after administration of MCFA's.
Figure 9 shows the lactic acid bacteria counts (as log₁₀ CFU) in the treated animals in the different parts of the gastrointestinal tract in function of time (in days) after administration of MCFA's.

### Example 1: Influence of MCFA on growth and survival of fungi and yeasts at pH 4 (mainly acid form of MCFA)

Six fungal/yeast strains were evaluated: Penicillium chrysogenum MUCL 28658, Aspergillus niger MUCL 19001, Candida albicans MUCL 29800, Fusarium oxysporum MUCL 781, Cephalosporum chrysogenum MUCL 9718 and Saccharomyces cerevisiae MUCL 31497. Each strain was incubated at 25 °C in P(otato)D(extrose) broth with following pH value and with following concentration of specific MCFA 50%/50% by weight (C₈-C₁₀): 2400 ppm (pH 4.0), 1200 ppm (pH 4.0), 600 ppm (pH 4.0), 300 ppm (pH 4.0), 0 ppm (pH 4.0), 0 ppm (pH 7.0). After 7 days, all fermentation broths were evaluated for growth. A plate count was performed in order to determine the type of inhibition (killing or static mode of action). Table 1 summarises the results for growth. At 300 ppm of the specific MCFA and for all microbial strains, no growth could be observed after plate count on PD agar.

**Table 1. Growth of fungi/yeasts at different concentrations of MCFA at pH 4.0 ("+" growth, "-" no growth).**

| | 0 ppm, pH 7 | 0 ppm, pH 4 | 300 ppm, pH 4 | 600 ppm, pH 4 | 1200 ppm., pH 4 | 2400 ppm, pH 4 |
|---|---|---|---|---|---|---|
| C. albicans | + | + | - | - | - | - |
| S. cerevisiae | + | + | - | - | - | - |
| A. niger | + | + | - | - | - | - |
| F. oxysporum | + | + | - | - | - | - |
| C. chrysogenum | + | - | - | - | - | - |
| P. chrysogenum | + | - | - | - | - | - |

From table 1, it is clear the specific MCFA (C₈-C₁₀) have an inhibiting effect at pH 4.0 on Aspergillus niger MUCL 19001, Candida albicans MUCL 29800, Fusarium oxysporum MUCL 781 and Saccharomyces cerevisiae MUCL 31497. The strains were killed, what caused growth inhibition. Penicillium chrysogenum MUCL 28658 and Cephalosporum chrysogenum MUCL 9718 did not grow at pH 4 (out of optimal pH range). For this reason, no conclusions are available for these two strains.

### Example 2: Influence of MCFA on growth and survival of fungi and yeasts at pH 7 (mainly salt form of MCFA)

The same experimental conditions were applied as described in experiment 1. However, in present experiment, all fermentation broths were set at pH 7.0. The results are summarised in table 2. Where strains did not grow, cells were also killed (verified by plate count on PD agar).

**Table 2. Growth of fungi/yeasts at different concentrations of MCFA at pH 7.0 ("+" growth, "-" no growth).**

| | 0 ppm, pH 7 | 300 ppm, pH 7 | 600 ppm, pH 7 | 1200 ppm, pH 7 | 2400 ppm, pH 7 |
|---|---|---|---|---|---|
| C. albicans | + | + | + | - | - |
| S. cerevisiae | + | - | - | - | - |
| A. niger | + | + | - | - | - |
| F. oxysporum | + | + | + | - | - |
| C. chrysogenum | + | + | - | - | - |
| P. chrysogenum | + | - | - | - | - |

From table 2, it can be concluded that use of specific MCFA (C₈-C₁₀) has an inhibiting effect on Penicillium chrysogenum MUCL 28658, Aspergillus niger MUCL 19001, Candida albicans MUCL 29800, Fusarium oxysporum MUCL 781, Cephalosporum chrysogenum MUCL 9718 en Saccharomyces cerevisiae MUCL 31497. Also here, the strains were killed, what results in growth inhibition.

From examples 1 and 2, it can be concluded that specific MCFA (C₈-C₁₀) can be applied as very effective antimicrobial agents for Aspergillus niger MUCL 19001, Candida albicans MUCL 29800, Fusarium oxysporum MUCL 781, Saccharomyces cerevisiae MUCL 31497, Penicillium chrysogenum MUCL 28658, Cephalosporum chrysogenum MUCL 9718. The antifungal effect is observed in a broad pH range.

### Example 3: In vivo evaluation of MCFA with early weaned piglets

The gastrointestinal tract of weaned piglets is very sensitive for Escherichia coli K88 contamination. Contamination with E. coli K88 mainly results in diarrhoea and lower performances (reflected in daily growth and feed conversion). In order to illustrate the control of E. coli K88 contamination in piglets by means of MCFA, following test was set up.

A mixture according to this invention was prepared which contained approximately 40 parts by weight of barley, 14 parts by weight of wheat, 10 parts by weight of maize products, 11 parts by weight of Soya products and 20 parts by weight of a feed supplement composition containing 0.8 parts by weight of specific MCFA with 8-10 carbon atoms. 10⁸ pathogenic bacteria (E. coli K88) were also added per g of feed.

A control feed was prepared which contained the same components as the above described mixture, with the exception that the control feed did not contain specific MCFA.

A group of 10 pigs have been weaned after a period of 21 days. All pigs had free access to water and feed. A first control group (group 1) was fed with the control feed. A second group (group 2) was fed with the feed composition of this invention as described above.

All animals were slaughtered 5 days after weaning. The number of bacteria per gram of stomach content was counted. The results are summarised in table 3.

**Table 3. Amount of bacteria (in log of counted amount) per g of stomach content.**

| | Group 1 | Group 2 |
|---|---|---|
| Pig 1 | 3.9 | 6.5 |
| Pig 2 | 2.5 | <1 |
| Pig 3 | <1 | <1 |
| Pig 4 | 7.6 | <1 |
| Pig 5 | 7.8 | <1 |

From table 3 it appears that by the addition of MCFA which contain 8-10 carbon atoms, bacteria are already killed in the stomach (acid conditions) of the animals. In 80 % of the piglets fed with the feed supplement of this invention, hardly any bacteria could be found in the stomach, whereas with the control feed in only 20% of the cases bacteria could be killed already in the stomach.

It is clear that supplementing a feed with one or more fatty acids containing 8-10 carbon atoms, the development of bacteria in the digestive tract of the young animal can be controlled. Most probably this effect can be explained by the fact that by the presence of these fatty acids in the stomach of the animal a physiological environment is created that is capable of regulating and stabilising the gastrointestinal microflora. Here, it has been found that fatty acids containing 8-10 carbon atoms are capable of killing the majority of the pathological bacteria (such as E. coli K88) already in the stomach, so that the transit of pathological doses of bacteria towards the intestines can be prohibited and the occurrence of gastrointestinal disorder prevented.

Moreover and as can be concluded from this example, MCFA are able to reduce E. coli contamination in a very complex environment, consisting of a very complex ecosystem and of a complex mixture of organic and inorganic matter, additives, aroma's, ....

### Example 4: Effect of specific MCFA on animal performances

The experiment disclosed in example 3 was repeated. It has further been found that the group of pigs fed with the feed composition of this invention showed an improved growth performance of approximately 7.5 % than the control group. At an age of 55 days, the mean weight of the piglets was approximately 19 kg. Such piglets are expected to reach the weight of 20 kg before day 60 of their life, which has been an objective that could not be reached for a long time. This means that the feed composition of this invention yields a higher daily growth. Moreover, since feed intake for both feeds was very similar, the feed composition of this invention results also in a lower feed conversion ratio.

In other words, the specific MCFA can be fed to animals in order to improve their performance (reflected in daily growth and feed conversion ratio), probably by regulating the microbial ecosystem of the gastrointestinal tract and by the fact that the MCFA are very quickly absorbed from the gastrointestinal tract and further converted to energy (which becomes available for the animal). This way, the specific MCFA can replace traditional growth promoters.

### Example 5: Comparison of MCFA and colistin for control of E. coli K88 growth

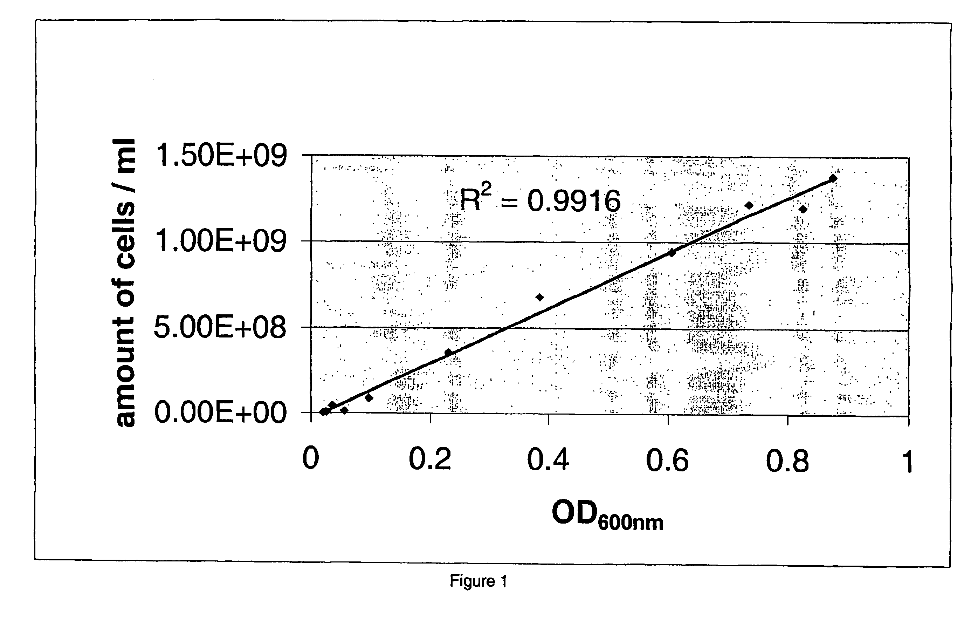

Three samples of 100 ml of fermentation broth (Brain Heart Infusion) were equally inoculated with an overnight culture of E. coli K88 (pathogen in gastrointestinal tract of piglet) and further incubated at 37°C. The optical density at 600 nm (OD₆₀₀ₙₘ) - which is proportional to the amount of E. coli cells present (figure 1) - was measured. As soon as an OD₆₀₀ₙₘ of between 0.2 and 0.5 was obtained,
(1) nothing was added to the first sample,
(2) 12 ppm of colistine was added to the second sample and
(3) 1200 ppm of the sodium salt of MCFA (mixture containing 50% of fatty acid with 8 carbon atoms and 50% of fatty acid with 10 carbon atoms) was added to the third sample.

The samples were further incubated at 37°C for 4 hours at pH 4.0. The OD₆₀₀ₙₘ was measured evey hour. The samples were removed from the incubation after 4 hours, the pH was measured in order to register possible pH changes during incubation. The results are summarised in Table 4.

**Table 4. Effect of MCFA and colistine (positive control) on E. coli K88**

| | OD₆₀₀ₙₘ | | |
|---|---|---|---|
| | Blank | Colistine | MCFA* |
| Hours after pH setting | | | |
| 2 | 0.478 | 0.420 | 1.259 |
| 3 | 0.491 | 0.449 | 1.276 |
| 4 | 0.558 | 0.455 | 1.262 |
| Difference in OD₆₀₀ₙₘ | 0.080 | 0.035 | 0.003 |
| Growth % | 100 | 44 | 4 |
| final pH | 4.04 | 3.93 | 4.28 |

| | | | |
|---|---|---|---|
| * Higher OD₆₀₀ₙₘ values by intrinsic turbidity caused by MCFA's | | | |

From table 4 it appears that the growth of E. coli is retarded for 56% by the addition of colistine and for 96% by the addition of MCFA of this invention. As the pH in the three samples was approximately the same, there is a very pronounced effect of the MCFA of this invention. It can be concluded that colistine, which is a traditional antibiotic, can be efficiently substituted by MCFA.

### Example 6: In vitro comparison of the antimicrobial activity of MCFA (50/50 C₈ and C₁₀) and commercially available antimicrobial agents.

The tests amounts vary dependent upon the technical information provided by the retailers of the known products. The object is to evaluate the antimicrobial activity of C₈/C₁₀ MCFA's and the commercially available CRINA® products (Akzo Nobel product) in the stomach. Four test substances were used:
a. Blank
b. MCFA's → 8 g / kg feed
c. CRINA® HC Piglets → 100 mg / kg feed
d. CRINA® HC 739 → 50 mg / kg feed
e. CRINA® HC Finishing Pigs/Sows → 75 mg / kg feed

The preparation of the test solution was as follows. Add to 1 kg feed (composition Table 5) an exactly determined quantity of *E*. *coli* K 88. Subsequently, create a suspension of 20% feed and 80% physiologic solution (0,85% saline). To simulate the 20%-suspension in a gastric environment, establish a pH of 3.5-4.0 with 0.1 N HCl.

**Table 5.**

| Experimental feed composition | |
|---|---|
| **Raw Material** | **Quantity (g)** |
| Wheat | 500 |
| Com, pressure cooked | 200 |
| Soya full fat beans Danex | 150 |
| Herringmeal | 50 |
| Whey powder | 70 |
| Biosow super | 30 |

The acidified suspension is now distributed in samples of 100 ml. A microbial count at t=0 was executed. Samples were incubated during 3h at 37° C. Samples were removed after 3h and pH and microbial count were determined. The *E. coli* strains were counted on a Coli ID medium (Bio Mérieux, 42017).

The planned observations were:
a. microbial count of control sample (blank) and treatments on t=0
b. microbial count of control sample (blank) and treatments on t=3h.

Table 6 depicts the calculated results proving the effectiveness of the antimicrobial effectiveness in the stomach of a MCFA (50/50 C₈/C₁₀) mixture according to the invention.

**Table 6.**

| Effect of 4 different treatments on *E. coli,* after 3 h of incubation at 37°C and pH 4.0. The values are expressed as log₁₀ CFU/ ml solution. | | | | |
|---|---|---|---|---|
| **Product** | ***E. coli* (t= 0h)** | ***E. coli* (t= 3h)** | Δ | Δ **to blank** |
| Blank | 7.3979 | 7.1972 | 0.2007 | 0.0000 |
| MCFA's | 7.4225 | 4.5303 | 2.8922 | 2.6669 |
| CRINA® HC Piglets | 7.3590 | 7.0105 | 0.3485 | 0.1867 |
| CRINA® HC 739 | 7.7887 | 7.0651 | 0.7236 | 0.1321 |
| CRINA® HC Finishing Pigs/Sows | 7.3652 | 6.5085 | 0.8567 | 0.6887 |

### Example 7: Antimicrobial effectiveness of MCFA-salts in a water solution and of a MCFA emulsion

The aim of this example is to study the effectiveness of a mixture of C₈ and C₁₀ (equal 50% by weight) MCFA's which is provided as a saline solution in the drinking water on the zoötechnical performance of chickens (growth, feed conversion ratio, feed intake, death).

Forty ROSS 308 cocks were housed in four different pens. Drinking water and feed were delivered at libitum. The test took 36 days. All chickens were given the same feed during the whole period. The feed does not contain any growth promoter nor coccidiostaticum. A mixture of C₈-C₁₀ was provided in the drinking water during the test period for pens 2, 3 and 4. Pen 1 was provided with pure drinking water, pens 2, 3 and 4 were provided with the MCFA's as salt in the following amounts, respectively: 0.5 kg, 1 kg and 2 kg per 1000 I.

The results of these tests are listed in tables 7, 8, 9, 10 and 11.

**Tabel 7.**

| Zoötechnical results of 0-12 days | | | | | |
|---|---|---|---|---|---|
| **Treatment** | **BW^{*2} (g)** | **DFI^{*1} (g/a/d)** | **BW^{*2} (g)** | **DWG^{*3} (g/a/d)** | **FCR^{*4}** |
| | **1 day** | **0-12 days** | **12 days** | **0-12 days** | **0-12 days** |
| 1 | 40.21 | 32.2 | 350.0 | 25.8 | 1.249 |
| 2 | 40.07 | 31.4 | 340.9 | 25.1 | 1.254 |
| 3 | 39.76 | 31.7 | 341.4 | 25.1 | 1.261 |
| 4 | 40.26 | 31.4 | 342.7 | 25.2 | 1.246 |

**Tabel 8.**

| Zoötechnical results of 12-23 days | | | | |
|---|---|---|---|---|
| **Treatment** | **DFI^{*1} (g/a/d)** | **BW^{*2} (g)** | **DWG^{*3} (g/a/d)** | **FCR^{*4}** |
| | **12-23 days** | **23 days** | **12-23 days** | **12-23 days** |
| 1 | 87.88 | 978.3 | 57.11 | 1.539 |
| 2 | 88.30 | 987.9 | 58.81 | 1.501 |
| 3 | 88.89 | 981.1 | 58.15 | 1.529 |
| 4 | 88.40 | 979.4 | 57.87 | 1.527 |

**Tabel 9.**

| Zoötechnical results of 23-28 days | | | | |
|---|---|---|---|---|
| **Treatment** | **DFI^{*1} (g/a/d)** | **BW^{*2} (g)** | **DWG^{*3} (g/a/d)** | **FCR^{*4}** |
| | **23-28 days** | **28 days** | **23-28 days** | **23-28 days** |
| 1 | 123.73 | 1349.0 | 74.14 | 1.669 |
| 2 | 104.52 | 1335.5 | 69.52 | 1.503 |
| 3 | 121.94 | 1364.5 | 76.70 | 1.590 |
| 4 | 122.76 | 1355.8 | 75.29 | 1.630 |

**Tabel 10.**

| Zoötechnical results of 29-36 days | | | | | |
|---|---|---|---|---|---|
| **Treatment** | **BW^{*2} (g)** | **DFI^{*1} (g/a/d)** | **BW^{*2} (g)** | **DWG^{*3} (g/a/d)** | **FCR^{*4}** |
| | **29 days** | **29-36 days** | **36 days** | **29-36 days** | **29-36 days** |
| 1 | 1452.2 | 242.2 | 2190.8 | 105.5 | 2.296 |
| 3 | 1498.5 | 245.4 | 2278.5 | 111.4 | 2.203 |

**Tabel 11.**

| Zoötechnical results of 0-36 days | | | | |
|---|---|---|---|---|
| **Treatment** | **DFI^{*1} (g/a/d)** | **BW^{*2} (g)** | **DWG^{*3} (g/a/d)** | **FCR^{*4}** |
| | **0-36 days** | **36 days** | **0-36 days** | **0-36 days** |
| 1 | 103.7 | 2190.8 | 59.74 | 1.736 |
| 3 | 104.6 | 2278.5 | 62.19 | 1.682 |

| | | | | |
|---|---|---|---|---|
| ^{*1} DFI: Daily Feed Intake | | | | |
| ^{*2}BW: Body Weight | | | | |
| ^{*3}DWG: Daily Weight Gain | | | | |
| ^{*4}FCR: Feed Conversion Ratio being the weight feed consumed per kg body weight gain. | | | | |

As can be seen from tables 7-11 during the first three weeks no specific differences were shown in these four populations. On the 36st day it is however clear that a higher body weight and a better FCR are obtained in the three pens in which the MCFA treatment is given. It is clear that the specific MCFA's composition according to the present invention have a positive effect on the zootechnical performance of chickens.

Another test was done in which the MFCA's (50/50 by weight C₈-C₁₀) were provided as an emulsion. When using higher concentrations up to several kg's of MCFA's in which these MCFA's were added to the drinking water as salt, foam formation could occur. In order to avoid these problems several anti-foaming agents could be added to the drinking water. However, the present invention found surprisingly that the specific fraction of MCFA's according to the invention could be better presented in the form of an emulsion. This approach avoided foam production.

Another test was performed wherein the salt form (1200 ppm) was compared with 50% MCFA emulsion (0.3 %) wherein a co-block polymer propylene-ethylene was used as emulsifying agent in xanthane and water. It is clear that other emulsifying agents may be chosen which are as such known in the art. Although the salt form resulted in an anti-microbacterial effect, the use of the emulsion was several times more active.

### Example 8: Use of MCFA's on pigs

The aim of this trial is to evaluate the effect of MCFA's (50%/50% by weight) in pig trials (from 30 to 105 kg).

The experimental farm is located in West-Flanders and consists of 2 bands, each containing 10 pens.

Each pen (2m x 4m) contains 14 pigs. Per pen, one feed supplier (*ad libitum*) is installed at the corridor's side. Water is supplied (*ad libitum*) on the other site of the pen. Gilts and boars are mixed. During the whole experiment, the pens are ventilated.

The feeds are distributed in such a way, that the average pig weight per feed is similar for all feeds at the beginning of the animal trial (± 30 kg). Following feeds were monitored during the trial:
**Feed 1:** control
**Feed 2:** control + 0,1 % MCFA's (growing phase)
   control + 0,05 % MCFA's (finishing phase)

Following parameters were followed: pig weight and feed uptake.

The pigs were weighed individually at '30 kg', '50 kg' and '100 kg'. The animal trial started on May 19, 2000. All pigs were weighed individually. The second weighing took place on June 26, 2000. The last weighing took place on September 25, 2000. Each pig was followed by its Sanitel number. Following summary can be derived from the obtained data (table 12):

**Table 12.**

| Growth of pigs receiving different diets | | |
|---|---|---|
| | **Weight at** | |
| **Diet 19/05/2000** | **26/06/2000** | **25/09/2000** |
| **(start)** | **(end growth phase)** | **(end finishing phase)** |
| 1 29,80 ± 5,65 | 51,64 ± 8,26 | 105,06 ± 15,30 |
| 2 29,94 ± 4,94 | 53,29 ± 7,68 | 104,87 ± 15,04 |

From table 12, it is clear that diet 2 results in the highest growth during the growing phase. At the end of the experience, this effect is disappeared. It seems that the MCFA's can be efficiently used as a growth promoter during the growing phase.

Feed uptake per pen was also registered. On June, 26, 2000, the original (growers) feed was changed and the pigs were further fed with finisher feed. Feed uptake per pig was measured for the growth phase and for the finishing phase (table 13). From these data, feed conversion ratio's could be further calculated (table 14).

**Table 13. Feed uptake of pigs receiving different diets**

| | Feed | uptake (kg) / pig |
|---|---|---|
| | Growth phase | Finishing phase |
| Diet 1 | 52,38 | 191 |
| Diet 2 | 53,64 | 180 |

**Table 14. Feed conversion ratio's of pigs receiving different diets**

| | Feed conversion | ratio |
|---|---|---|
| | Growth phase | Finishing phase |
| Diet 1 | 2,40 | 3,46 |
| Diet 2 | 2,30 | 3,36 |

From tables 13 and 14, it is clear that supplementation of MCFA's to the feed results over the complete period in a lower feed conversion ratio. While MCFA's has not any effect on growth, it has a positive effect on feed conversion ratio during finishing phase.

In conclusion, MCFA's can be efficiently used as "growth promoter" during growth phase, while this effect disappears later on.

### Example 9: MIC value determination of MCFA's

This test was subcontracted to University Gent

A trial was carried out to determine the in vitro susceptibility to the MCFA's of different collection strains of bacteria commonly associated with the intestinal tract. Minimal inhibitory concentrations (MIC) of the product on collection strains varied from 0.25% to 0.005%. At pH 7.2, the highest activity was seen on enterococci grown anaerobically at low pH. Lowest activity was noted on *Enterobacteriaceae* and on *Pseudomonas* grown aerobically.

Seventeen collection strains representing various facultatively aerobically growing intestinal bacteria were included in the study (Table 15). Three antibiogram control strains *S. aureus* ATCC 25922, *Enterococcus faecalis* ATCC 29212 and *E*. *coli* 25923 were tested as well.

Three tenfold dilutions of a 10% suspension of the product were prepared in distilled water. To obtain the desired final concentrations, 1.5, 0.75 and 0.36 ml of these dilutions were pipetted in petri dishes and carefully mixed with molten growth media. Three media were used Mueller - Hinton II medium (BBL) with a pH of 7.2, MRS medium (Oxoid) plates with pH 6.2 and MRS medium with pH adjusted to 4.6.

Preserved strains were grown on Columbia blood agar plates or MRS medium. Inocula were prepared from overnight 16 to 26 h old broth cultures incubated at 37°C. These were obtained by suspending growth in sterile saline in a photometer adapted for McFarland scale measurements (bioMérieux). Solutions matching 0.5 McFarland were diluted 10-fold in saline and inoculated on the antibiotic and control plates using a Denley Multipoint Inoculator (Mast). This way, approximately 10,000 colony forming units of each strain was inoculated on the plates. The MRS plates were incubated anaerobically in a H₂ + CO₂ atmosphere. The Mueller-Hinton plates were incubated aerobically.

Readings were performed after incubation at 37°C for 2 days. The MIC was recorded as the lowest concentration that completely or nearly completely inhibited growth, thus disregarding faint hazes of growth or single colonies.

Minimal inhibitory concentrations are shown in Table 1. Only the four *Lactobacillus* strains grew very well at pH 4.6. The growth of most of the enterococcal strains was fairly well developed at this pH but still less than on the other media. The remaining strains did not grow. All strains except the Gram-negative ones grew on MRS at pH 6.2. The lactobacilli developed to only faint hazes of growth on Mueller-Hinton agar.

The MICs of all isolates were all situated between 0.25% and 0.0025% MCFA-emulsion. The product was most active at pH 4.6 on the enterococci and lactobacilli that were able to grow. In conditions for optimal in vitro growth the MIC varied between 0.25 and 0.1%

**Table 15.**

| *In vitro* inhibitory activity of MCFA's on bacterial strains | | | | |
|---|---|---|---|---|
| | | | | |
| Species | Strain No | Minimal inhibitory concentration (%) on | | |
| | | | | |
| | | MRS pH 4.6 | MRS pH 6.2 | Mueller- Hinton |
| | | | | |
| *Bacillus cereus* | 01/3 | No growth | No growth | 0.1 |
| *Staphylococcus aureus* | ATCC 22923 | No growth | No growth | 0.01 |
| *Enterococcus faecium* | 23a | 0.01 | 0.1 | 0.1 |
| *Enterococcucs faecium* | 18a | 0.01 | 0.1 | 0.1 |
| *Enterococcus faecium* | LMG 11423 | 0.005 | 0.1 | 0.1 |
| *Ertterococcus faecalis* | 19b | 0.005 | 0.1 | 0.1 |
| *Enterococcus faecalis* | ATCC 29212 | No growth | 0.1 | 0.1 |
| *Enterococcus faecalis* | 13a | No growth | 0.1 | 0.1 |
| *Enterococcus cecorum* | B0028-11 | No growth | 0.05 | 0.1 |
| *Enterococcus cecorum* | B0027-0 | No growth | 0.05 | 0.1 |
| *Enterococcus hirae* | 74 | 0.005 | 0.1 | 0.1 |
| *Enterococcus hirae* | 79 | 0.005 | 0.1 | 0.1 |
| *Pseudomonas aeruginosa* | 01/350 | No growth | No growth | 0.25 |
| *Escherichia coli* | ATCC 25922 | No growth | No growth | 0.25 |
| *Salmonella* ser. typhimurium | DAB76 | No growth | No growth | 0.25 |
| Salmonella ser. typhimurium | DAB75 | No growth | No growth | 0.25 |
| *Lactobacillus delbrueckii* | LAB 043 | 0.025 | 0.1 | Faint growth |
| *Lactobacillus coryniformii* | LAB 34 | 0.025 | 0.1 | Faint growth |
| *Lactobacillus gasseri* | LAB 060 | 0.025 | 0.1 | Faint growth |
| *Lactobacillus cellobiosus* | LAB 031 | 0.025 | 0.1 | Faint growth |

### Example 10: Effectiveness of MCFA's on piglets (7-20 kg)

Effectiveness on the *Enterobacteriaceae* will be the object of example 10. The antimicrobial activity of a 50% MCFA emulsion will be evaluated by using a population of piglets (7-20 kg).

Early weaned pigs (7 kg) are housed in two populations of each ten specimen. Water and feed were provided ad libitum. Population (C) was provided with a basic feed which does not contain antibiotica nor growth promoters.

Population (B) was given basic feed supplemented with 0.50 % 50% emulsion of MCFA (50/50 C₈-C₁₀, with emulsifying agent co-block polymer ethylenepropylene). For each sample two times two animals were selected from the control and the treated group and were dissected according to approved ethical proceedings. The contents collected from several parts of the gastrointestinal tract being the stomach, duodenum, ileum and the first part of the caecum. Two mixed samples per gastrointestinal tract locus of respectively control and treatment were made and analysed on four different media: nutrient agar for the count of the total contamination, violet red bile glucose agar for the count of the enterobacteria, coli ID for the count of the E. coli and Rogosa agar for lactic acid bacteria.

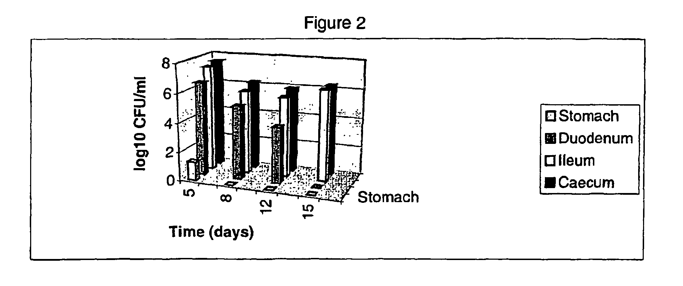

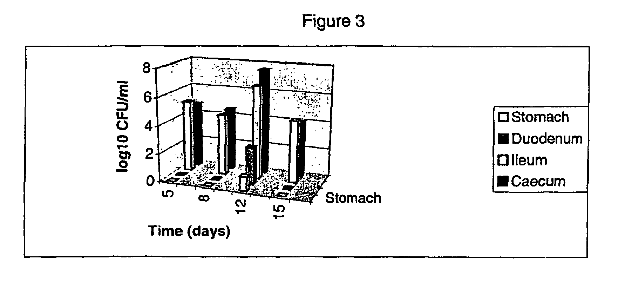

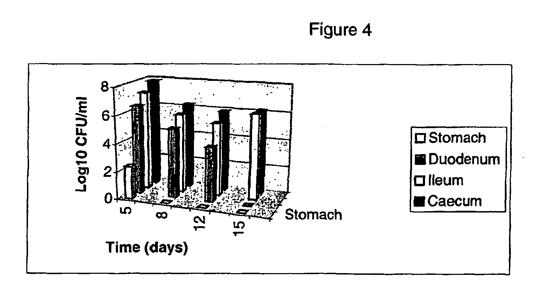

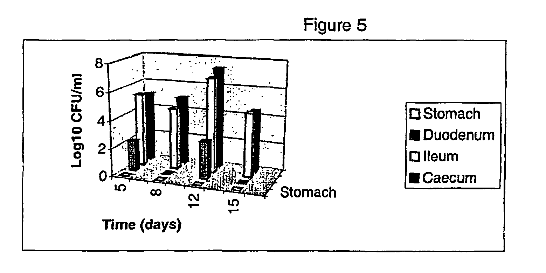

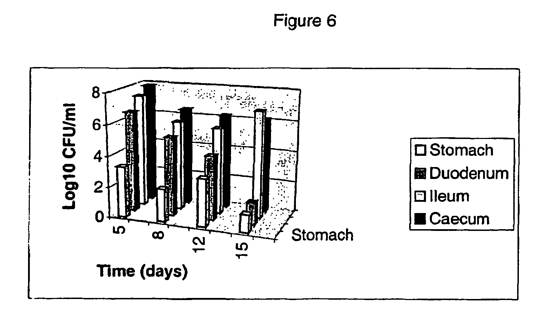

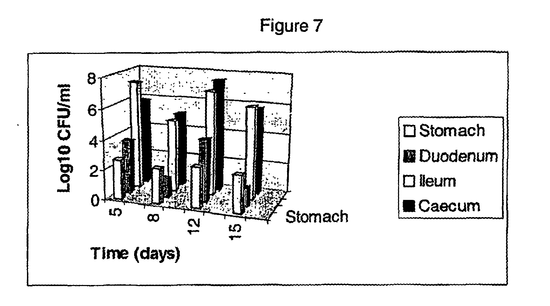

The results of these different counts are depicted in figures 2-9. It is clear that the population of E. coli and enterobacteria reduced, in particular in the first part, which is the most acidic part of the gastrointestinal tract.

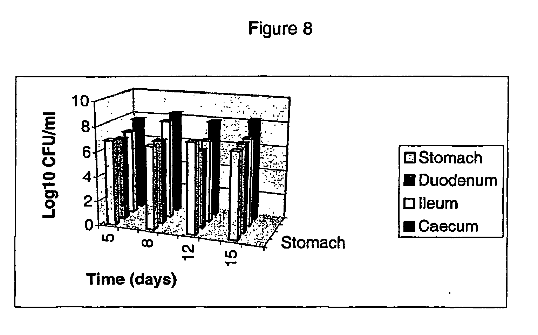

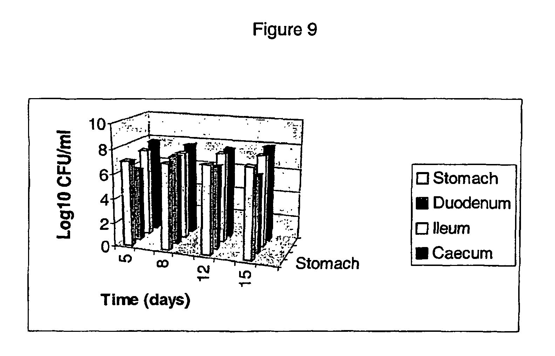

Figures 8 and 9 show the surprising selective effect on microbial organisms. The population of unwanted and possible pathogenic species such as E. coli, enterobacteria is diminished substantially. The population of wanted species such as the lactic acid bacteria remains unaffected.

It is clear that the examples are only illustrative and non-limiting for the invention. A possible range of weight % of a feed supplement according to the invention in a mixture of 20-50% C₈, 20-50% C₁₀ and any other MCFA from C₆-C₂₄ up to 100% could be suitable.

Several of these feed supplements were tested and performed similar as the standard 50%/50% C₈-C₁₀ composition used in the cited examples.

The surprising selective action against specific species was also present using the latter ranges.

### References

BARTON, M.D. (1998). Does the use of antibiotics in animals affect human health. Aust. Vet. J., 76, 177.
BARTOV, I., PASTEUR, N. AND USHER, N. (1982). The nutritional value of moldy grains for broiler chicks. Poult. Sci., 61, 2247.
DUPONT, H.L. AND STEELE, J.H. (1987). Use of antimicrobial agents in feeds: implications for human health. Rev. Infect. Dis., 9, 447.
ECKEL, B. (1999). Probiotics can improve intestinal microbe balance and feed hygiene. Feed Tech., 3, 39.
EMELE, F.E., FADAHUNSI, A.A., ANYIWO, C.E. AND OGUNLEYE, O. (2000). A comparative clinical evaluation of econazole nitrate, miconazole and nystatin in the treatment of vaginal candidiasis. West Afr. J. Med., 19, 12.
ENGLISH, P.R., TOPPS, J.H. AND DEMPSTER, D.G. (1973). Moist barley preserved with propionic acid in the diet of pigs. Anim. Prod., 17, 75.
FOSTER, B.C., TRENHOLM, H.L. AND FRIEND, D.W. (1987). The effect of a propionate feed preservative in deoxynivalenol (Vomitoxin) containing corn diets fed to swine. Can. J. Anim. Sci., 67, 1159.
GUILLOT, J.F. (1989). Apparition et evolution de la resistance bacteriènne aux antibiotiques. Ann. Rech. Vét., 20, 3.
HAMILTON, P.B. (1990). Problems with mycotoxins persist but can be lived with. Feedstuffs, January 22, 22.
HARVEY, R.B., HUFF, W.E., KUBENA, L.F. AND PHILLIPS, T.D. (1989). Evaluation of diets contaminated with aflatoxin and ochratoxin fed to pigs. Am. J. Vet. Res., 50, 1400.
HIROOKA, E.Y., YAMAGUCHI, M.M., AOYAMA, S., SUGIURA, Y. AND UENO, Y. (1996). The natural occurrence of fumonisins in Brazilian corn Kernels. Food Addit. Contam., 13, 173.
JELINEK, C.F., POHLAND, A.E. AND WOOD, G.E. (1989). Worldwide occurrence of mycotoxins in foods and feeds - an update. J. Assoc. Off. Anal. Chem., 72, 223.
JONES, G.M., DONEFER, E. AND ELLIOT, J.I. (1970). Feeding value for dairy cattle and pigs of high-moisture corn preserved with propionic acid. Can. J. Anim. Sci., 500, 483.
KABARA, J. (1978). Fatty acids and derivatives as antimicrobial agents - a review. In: KABARA, J. (ed.). The pharmaceutical effects of lipids, AOCS, Champaign, 111, USA, 1.
MARIN, S., ANCHIS, V., SANZ, D., CASTEL, I., RAMOS, A.J., CANELA, R. AND MAGAN (1999). Control of growth and fumosin B1 production by Fusarium verticillioides and Fusarium proliferatum isolates in moist maize with propionate preservatives. Food Addit. Contam., 16, 555.
MUIRHEAD, S. (1998). EU ban of antibiotics draws sharp criticism. Feedstuffs, 70, 1. PIER, A.C. (1992). Major biological consequences of aflatoxicosis in animal production. J. Anim. Sci., 70, 3964.
PRESCOTT, J.F. (1997). Antibiotics: miracle drugs or pig food. Can. Vet. J., 38, 763. RAHNEMA, S. AND NEAL, S.M. (1992). Preservation and use of chemically treated high-moisture corn by weanling pigs. J. Prod. Agric., 5, 458.
RAHNEMA, S. AND NEAL, S.M. (1994). Laboratory and field evaluation of commercial feed preservatives in the diet of nursery pigs. J. Anim. Sci., 72, 572.
REISS, J. (1978). Mycotoxins in foodstuffs. XI. Fate of aflatoxin B1 during preparation and baking of whole wheat bread. Cereal Chem., 55, 421.
ROSS, I.W. (1999). Antibiotics in animal feed stocks and implications of the European Commission ban. Food Test Anal., 5, 8.
RUSSELL, L., COX, D.F., LARSEN, G., BODWELL, K. AND NELSON, C.E. (1991). Incidence of molds and mycotoxins in commercial animal feed mills in seven midwestern states. J. Anim. Sci., 69, 5.
RYBINSKA, K., POSTUPOLSKI, J. AND SZCZESNA, M. (1997). Determination of natamycin residues in ripening cheese by performance liquid chromatography. Rocz. Panstw. Zakl. Hig., 48, 173.
SMITH, P.A., NELSON, K.L., KIRBY, Z.B., JOHNSON, Z.B. AND BEASLEY, J.N. (1983). Influence of temperature, moisture and propionic acid on mold growth and toxin production on corn. Poult. Sci., 62, 419.
TUITE, J. (1979). Field and storage conditions for the production of mycotoxins and geographic distribution of some mycotoxin problems in the United States. In: Interactions of mycotoxins in animal production. National Academy of Sciences, Washington.
WYATT, R.D. (1995). Molds, mycotoxins, and the problems they cause. Alltech symposium.
YASIN, M. AND HANNA, M; (1989). Potassium sorbate as a preservative for high moisture corn. Rans. Am. Soc. Agric. Eng., 32, 280.
ZIJLSTRA, R.T. WANG, K.Y., EASTER, R.A. AND OLDE, J. (1996). Effect of feeding a milk replacer to early-weaned pigs on growth, body composition, and small intestinal morphology, compared with suckled littermates. J. Anim. Sci., 74, 2948.

## Claims

1. Use of two medium chain fatty acids (MCFA), chosen from the group consisting of caprylic (C₈) acid and capric (C₁₀) acid, emulsions or mixtures thereof, about equal amounts by weight, in an amount of:
20-50% C₈
20-50% C₁₀
and optionally other MCFA chosen from C₆- C₂₄
wherein the MCFA concentration is 100-3000ppm for the manufacture of a medicament for the inhibition of microbial contamination, growth and/or the subsequent toxin production.

2. Use according to claim 1, of caprylic (C₈) acid and capric (C₁₀) acid in about equal amounts by weight and in a concentration of 100-3000ppm for the manufacture of a medicament for the inhibition of microbial contamination, growth and/or the subsequent toxin production.

3. Use according to claim 1 or 2 , in combination with other MCFA, such as lauric (C₁₂) and myristic (C₁₄) acid, other antifungal agents or with other (organic) (fatty) acids or with additives, such as aroma's and plant extracts.

4. Use according to any of the previous claims 1 to 3, for combatting the growth of fungi and yeasts chosen from the group: Penicillium, Aspergillus, Fusarium, Cephalosporum, Saccharomyces, Candida as well as to other Fungi Imperfecti and Hemiascomycetes (yeasts).

5. Use according to any of the previous claims 1 to 4, for selective combatting the growth of Gram negative bacteria such as Escherichia coli, Salmonella sp., Shigella sp. and other Gram negative and coliform bacteria and food/feed spoilers.

6. Use according to any of the previous claims 4 or 5, for the selective control and regulation of the microbial ecosystem in the gastrointestinal tract of any animal or human.

7. Use according to claim 6, wherein the animals are in their early weaning phase.

8. Feed composition for an animal comprising a feed supplement containing one or more medium chain fatty acids (MCFA) chosen from the group consisting of caprylic (C₈) acid and capric (C₁₀) acid, emulsions or mixtures thereof in an amount by weight % of:
20-50% C₈
20-50% C₁₀
and optionally other MCFA chosen from C₆-C₂₄,
wherein the MCFA concentration is 100-3000ppm.

9. Feed composition according to claim 8, containing caprylic (C₈) acid and capric (C₁₀) acid in about equal amounts by weight and in a concentration of 100-3000ppm.

## Patentansprüche

1. Verwendung von zwei Fettsäuren mittlerer Kettenlänge (MCT-Fette), die gewählt sind aus der Gruppe bestehend aus Caprylsäure (C₈) und Caprinsäure (C₁₀), Emulsionen oder Mischungen davon, gewichtsmäßig ungefähr gleiche Mengen, in Mengen von:
20 - 50 % C₈,
20 - 50 % C₁₀,
und wahlweise anderen Fettsäuren mittlerer Kettenlänge, gewählt aus C₆-C₂₄,
wobei die Konzentration der MCT-Fette 100 - 3000 ppm ist,
für die Herstellung eines Medikaments zur Hemmung von mikrobieller Kontamination, mikrobiellem Wachstum und/oder darauffolgender Toxinproduktion.

2. Verwendung nach Anspruch 1 von gewichtsmäßig ungefähr gleiche Mengen Caprylsäure (C₈) und Caprinsäure (C₁₀), wobei die Konzentration 100 - 3000 ppm ist, für die Herstellung eines Medikaments zur Hemmung von mikrobieller Kontamination, mikrobiellem Wachstum und/oder darauffolgender Toxinproduktion.

3. Verwendung nach den Ansprüchen 1 oder 2, in Kombination mit anderen MCT-Fette, wie Laurinsäure (C₁₂) und Myristinsäure (C₁₄), anderen Mitteln gegen Pilze oder mit anderen organischen (fett-) Säuren oder mit Zusatzstoffen, sowie Aromen und Pflanzenextrakten.

4. Verwendung nach einem der vorhergehenden Ansprüche 1 bis 3, für die Bekämpfung des Wachstums von Pilzen und Hefen aus der Gruppe: Penicillium Aspergillus, Fusarium, Cephalosporum, Saccharomyces, Candida wie auch anderer Fungi Imperfecti and Hemiascomycetes (Hefen).

5. Verwendung nach einem der vorhergehenden Ansprüche 1 bis 4, für die selektive Bekämpfung des Wachstums gramnegativer Bakterien wie Escherichia coli, Salmonella sp., Shigella sp. und anderer gramnegativer und coliformer Bakterien und Nahrungs- und Futtermittel verderbender Bakterien.

6. Verwendung nach irgendeinem der vorhergehenden Ansprüche 4 oder 5, für die selektive Kontrolle und Regulierung des mikrobiellen Ökosystems im Gastrointestinaltrakt eines Tiers oder Menschen.

7. Verwendung nach Anspruch 6, wobei die Tiere in ihrer frühen Entwöhnungsphase sind.

8. Futtermittelzusammensetzung für ein Tier, das einen Futterzusatz umfasst, der eine oder mehrere Fettsäuren mittlerer Kettenlänge (MCT-Fette) enthält, die gewählt sind aus der Gruppe bestehend aus Caprylsäure (C₈) und Caprinsäure (C₁₀), Emulsionen oder Mischungen davon, in einem Gewichtsverhältnis von:
20 - 50 % C₈
20 - 50 % C₁₀
und wahlweise anderen MCT-Fette, gewählt aus C₆-C₂₄,
wobei die Konzentration der MCT-Fette 100 - 3000 ppm ist.

9. Futtermittelzusammensetzung nach Anspruch 8, das Caprylsäure (C₈) und Caprinsäure (C₁₀) umfasst in gewichtsmäßig ungefähr gleiche Mengen, wobei die Konzentration 100 - 3000 ppm ist.

## Revendications

1. Utilisation de deux acides gras à chaîne moyenne (AGCM) choisis parmis le groupe qui consiste de l'acide caprylique (C₈) et l'acide caprique (C₁₀), les émulsions ou les mélanges correspondants, en quantité environ égale par poids, et en quantité de
20 à 50 % de C₈
20 à 50 % de C₁₀,
et éventuellement d'autres AGCMs choisis parmis le groupe C₆-C₂₄,
**caractérisée en ce que** la concentration des AGCMs est 100 - 3000 ppm, pour la fabrication d'un médicament pour l'inhibition de la contamination microbienne, sa croissance et/ou la production ultérieure de toxines.

2. Utilisation selon la revendication 1 de l'acide caprylique (C₈) et l'acide caprique (C₁₀) en quantité environ égale par poids et ayant une concentration de 100 - 3000 ppm pour la fabrication d'un médicament pour l'inhibition de la contamination microbienne, sa croissance et/ou la production ultérieure de toxines.

3. Utilisation selon les revendications 1 ou 2, en combinaison avec d'autres AGCMs, tels que l'acide laurique (C₁₂) et l'acide myristique (C₁₄), d'autres agents antifongiques, ou avec d'autres acides (gras) (organiques), ou avec des additifs, tels que des arômes et des extraits de plantes.

4. Utilisation selon l'une quelconque des revendications 1 à 3 précédentes pour lutter contre la croissance des champignons et des levures choisis parmis le groupe : Penicillium, Aspergillus, Fusarium, Céphalosporum, Saccharomyces, Candida ainsi que d' autres Fungi Imperfecti et Hémiascomycètes (levures).

5. Utilisation selon l'une quelconque des revendications 1 à 4 précédentes pour lutter sélectivement contre la croissance des bactéries Gram négatives telles que Escherichia coli, Salmonella sp., Shigella sp., et autres bactéries Gram négatives et coliformes et contaminateurs de nourriture/aliments des animaux.

6. Utilisation selon l'une quelconque des revendications 4 ou 5 précédentes pour le contrôle et la régularisation sélective de l'écosystème microbien du tractus gastro-intestinal animal ou humain.

7. Utilisation selon la revendication 6, **caractérisée en ce que** les animaux se trouvent en début de sevrage.

8. Composition alimentaire pour un animal qui comprend un supplément alimentaire qui contient un ou davantage d'acides gras à chaîne moyenne (AGCM) choisis parmis le groupe qui consiste de l'acide caprylique (C₈) et l'acide caprique (C₁₀), les émulsions ou les mélanges correspondants, en quantité par % de poids, de
20 à 50 % de C₈
20 à 50 % de C₁₀,
et éventuellement d'autres AGCMs choisis parmis le groupe C₆-C₂₄
**caractérisée en ce que** la concentration des AGCMs est 100 - 3000 ppm.

9. Composition alimentaire selon la revendication 8, qui comprend de l'acide caprylique (C₈) et l'acide caprique (C₁₀) en quantité environ égale par poids et ayant une concentration de 100 - 3000 ppm.
